# EUROPEAN PATENT SPECIFICATION

(11) **EP 0 700 668 B1**
(45) Date of publication and mention of the grant of the patent: **13.12.2000**
(21) Application number: 94306675.3
(22) Date of filing: 12.09.1994
(51) Int. Cl.: A61F 2/00, F16K 31/08

(54) **Intraurethral magnetic valve**
Intraurethralmagnetventil
Valve magnétique intraurétrale

(43) Date of publication of application: 13.03.1996
(73) Proprietor: Davis, Phillip J., New York, New York 10024 (US)
(72) Inventor: Davis, Phillip J., New York, New York 10024 (US)
(74) Representative: Feakins, Graham Allan

(56) References cited:
- US-A- 3 731 670
- US-A- 4 994 019
- US-A- 5 135 538
- US-A- 5 140 999
- US-A- 5 366 506

## Description

This invention relates to intraurethral magnetic valves that are used to provide a valving means for individuals suffering from an inability to control their bladder functions by substituting a mechanical magnetic actuated valve to control the flow of urine.

Known devices of this type have relied on a wide variety of different magnetic valve configurations that attempt to control the fluid flow from the bladder. Examples of these can be divided into two basic designs. Firstly, there are ferromagnetic spheres or slugs which seal an aperture by attraction to one or more fixed permanent magnets within a valve housing and are drawn away from the aperture by a strong permanent magnet or electromagnetic field induced by a hand held magnet located outside the valve housing, see for example US-A-3,731,670 and US-A-5,004,454.

The second group is characterised by permanent magnets bonded to nonmagnetic check valves which are drawn to seal an aperture by attraction to a ferromagnetic element comprising all or part of the aperture or are resiliently held in a closed position on the aperture by a spring. These checks are drawn in a straight linear motion away from the apertured valve seat by a hand held magnet outside the valve housing, see for example US-A-3,812,841 and US-A-5,140,999.

In US-A-3,731,670 a corporeal fluid control using a binary magnetic duct valve is disclosed wherein a mounting tube has two spaced magnets with a steel ball valve element therebetween. The ball valve element is selectively attracted to each of the magnets defining an open or closed state depending on which magnet the ball is engaged. The ball is reciprocated within the structure by an external magnetic force.

US-A-5,004,454 is directed to an auxiliary intraurethral magnetic valve wherein a plastics tube defines the valve body and a valve seat. A valve element of a ferromagnetic material is held in sealing relation against the valve seat by a spring associated therewith. The valve is opened by imposing a magnetic force on the valve element drawing same away from the seat and stretching the spring.

The first type of device tends to suffer from an intrinsic difficulty in balancing the magnetic force needed for adequate sealing with the need for reasonable operational range of the activation magnet.

Since magnetic forces are highly nonlinear, decreasing very rapidly with distance from the magnetic poles; when a ferromagnetic ball or slug is held against an aperture by a permanent magnet element the respective switching magnet must be either very large or very close to induce the required force. The slug checks that are drawn to the side of the housing encounter high sidewall friction forces which are induced increasingly as the angle between the housing centre line and the line of approach of the switching magnet increases.

In US-A-3,812,841 a urethra magnetic valve structure has a valve element positioned on a movable magnetic core cylinder. The valve element is held in closed position by an attached spring. Inducing a high electromagnetic force from outside the body will move the magnetic core cylinder and valve element attached thereto opening the valve.

In US-A-3,812,841, the valve check movement is constrained by the housing to a straight line which necessitates that unless the switching magnet approaches with its axis directly aligned on the centre line the force will draw the check against the sidewall imparting torque induced friction decreasing the effectiveness and movement of the check within the valve housing.

US-A-5,140,999 is directed to an implantable valve structure in which the valve element extends well within the bladder for increased lateral operational movement. The valve element has a compression spring engaging same in a closed or checked position. Upon inducement of an outside magnetic force the magnetisable member on the free end of the valve element within the bladder is displaced to the side moving the respective valve element off its valve seat opening the valve. No accommodation is made for bladder neck movements or changes in bladder inflation which may cause unwanted movements of the free end of the valve element.

It is known from US-A-3731670 to provide an intraurethral magnetic valve comprising a tubular housing of non-magnetic material, a valve element within said housing and an apertured valve seat sealingly engageable by said valve element, said valve element being normally magnetically attracted to said valve seat thereby occluding and sealing same and there being separate magnetic means for opening said valve by imposing a magnetic field on said valve element to overcome the magnetic attraction to said valve seat thereby moving said valve element away from sealing relation with said valve seat.

The invention is characterised over the disclosure of US-A-3731670 in that said valve element comprises a permanent magnet (19, 29, 46) and a non-ferromagnetic means (18B, 30, 47) for spacing said permanent magnet with respect to said valve seat, and wherein said moving said valve element away is pivoting said valve element away.

For a better understanding of the invention, and to show how the same may be carried into effect, reference will now be made by way of example, to the accompanying drawings, in which:-
Figure 1 is a sectional view taken through an intraurethral magnetic valve shown within a placement catheter;
Figure 2 is a section on lines 2-2 in Figure 1;
Figure 3 is a sectional view of an alternative form of the valve, in a closed position;
Figure 4 is a sectional view of the form shown in Figure 3 in an open position;
Figure 5 is a section on lines 5-5 in Figure 3;
Figure 6 is a section on lines 6-6 in Figure 3;
Figure 7 is a side view with portions in sections showing a catheter placement device for the valve;
Figure 8 is a diagrammatic sectional view showing the device implanted for controlling flow through the urethra; and
Figure 9 is a sectional view of another form of the device, showing an encapsulated magnet.

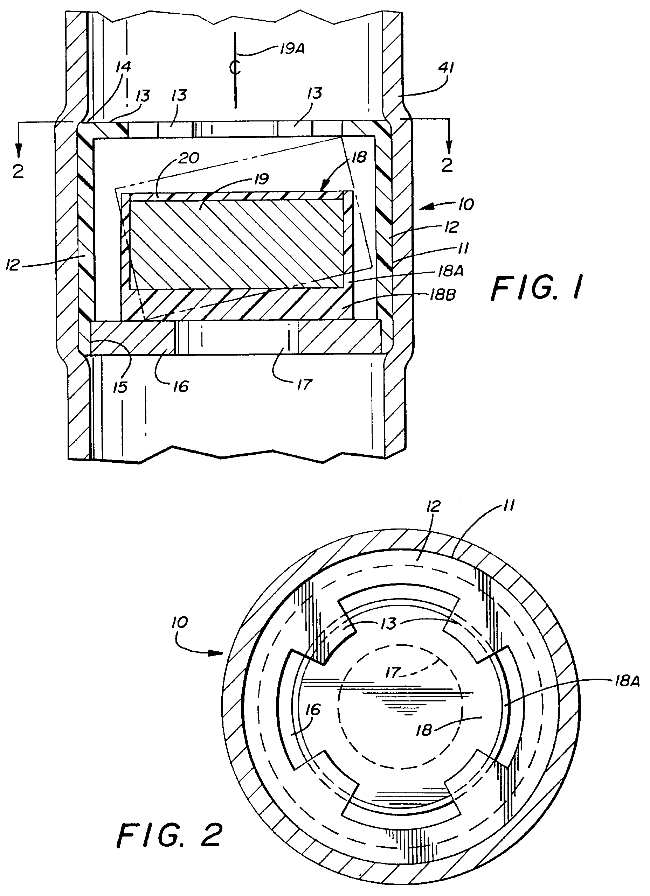

Referring to Figures 1 and 2 of the drawings, an intraurethral relief valve assembly 10 comprises a cylindrical non-magnetic housing 11 having a continuous side wall 12. A plurality of annularly spaced retaining flanges 13 extend inwardly from a perimeter edge 14 of the cylindrical housing 11 defining the sidewall 12. A recessed area 15 in the sidewall 12 extends inwardly from its free end defining a mounting surface for registration with a ferromagnetic valve seat 16. The valve seat 16 has a cylindrical aperture at 17 concentric within the cylindrical housing 11. A valve element 18 comprises a cylindrical magnet 19 encapsulated within a generally U-shaped non-magnetic capsule 18A of biocompatible material having a thicker base portion 18B adjacent the valve seat 16 and an oppositely disposed closer 20 adhesively fixed to the magnet 19.

The valve element 18 is normally attracted to the ferromagnetic valve seat 16 for sealing registration therewith occluding the cylindrical aperture at 17, thereby closing the valve 10. The magnet 19 is magnetised along its central line axis.

The flanges 13 may also be used as engagement points for gripping and positioning the valve assembly 10 within the body lumen or placement catheter.

It is important that the magnet 19 has high coercive force and high residual flux density to achieve the proper durability and sealing ability.

Figure 1 shows the valve assembly 10 in the closed position with the valve element 18 shown in broken lines indicating the free range of travel required during operation and thus the relative clearance of the valve element 18 within the housing 11 when open.

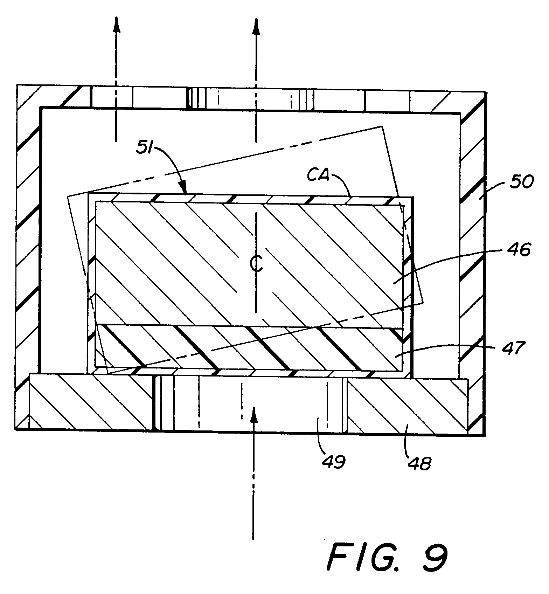

This valve open position occurs upon activation of the valve element 18 by a switching magnet 21 (Figure 8). The magnet 21 is of a generally bar configuration in this example, having been magnetised along its longitudinal centre line axis A. When the magnet 21 is brought into close proximity to the valve element 18 as indicated with their respective magnetic centre lines in a non-axial alignment, magnetic torque is induced on the valve element 18. The misalignment of the respective magnetic fields of the switching magnet 21 and the magnet 19 within the valve element 18 induces the rotation of the valve element 18 to reposition within the housing 11 as shown in broken lines in Figure 1, thus opening the valve assembly 10 for passage of fluid. The valve element 18 should be of a sufficient size to prevent excess rotation that would and could lead to a potential to jam or impinge against the inner surface of the housing 11, thus preventing reseating of the valve element 18 in the aperture occluding position by the limited magnetic attraction available.

The retaining flanges 13 limit the relative movement of the valve element 18 within the housing so that the required range of magnetic attraction between the valve element 18 and the valve set 16 is sufficient to return the valve element 18 to the aperture occluding position upon deactivation i.e. removal of the switching magnet 21 (as shown in solid lines in Figure 1).

The valve element 18 also acts as a high pressure relief element, with relief pressure determined by the magnitude of the attraction forces between the valve element 18 and valve seat 16 at the relative separation distance imposed by the non-ferromagnetic base portion 18B of the capsule 18A. The axial symmetry of the valve seat 16 and the magnet 19 within the valve element 18 limits magnetic attractive forces that tend to draw the valve element 18 against the inner walls of the housing 11 and thus limits friction between the housing 11 and valve element 18. This symmetry also provides uniform distribution of closure force around cylindrical aperture at 17 in the valve seat 16. Relief pressure may be altered by varying any combination of the non-ferromagnetic capsule base 18B thickness, the mass of the valve seat 16, the magnetic permeability of the valve seat 16, or the mass of the magnet 19, its respective magnetic aspect ratio (length to diameter) or its magnetic residual flux density in the valve element 18, and the aperture area at 17 of the valve seat 16.

The switching magnet 21 may be positioned at any angle relative to the valve element 18 and enclosed magnet 19 centre line axis indicated at 19A.

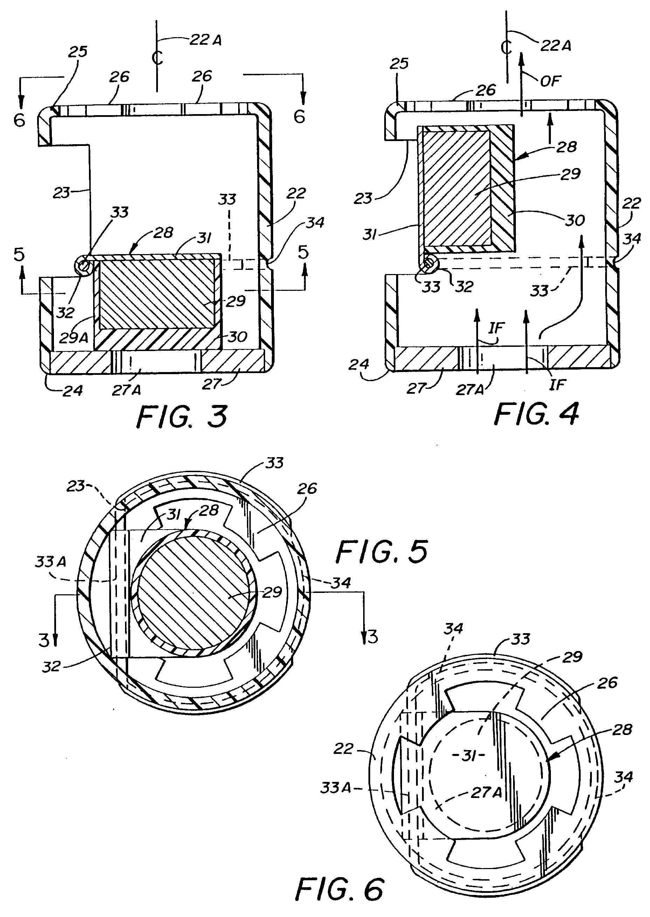

Referring now to Figures 3-6, an alternative form is shown wherein a cylindrical valve housing 22 has a cut through its sidewall at 23 inwardly of its respective ends 24 and 25. Retaining flanges 26 and valve seat 27 apertured at 27A are identical to those described above.

A hinged valve element 28 is of a similar construction as that of said valve element 18, having an encapsulated magnet 29 adhesively bonded within a biocompatible material and a non-ferromagnetic capsule 29A characterised by a thicker base portion 30.

The hinged valve element 28 has a mounting plate closure 31 that is secured to the encapsulated magnet 29 opposite the base portion 30. The mounting closure plate 31 extends to form a knuckle 32 configured thereon for pivotal registration of a hinge pin 33 that extends through the knuckle 32 and around the exterior surface of the cylindrical valve housing 22 in a mounting groove 34, best seen in Figures 5 and 6. The hinged valve element 28 is normally in the closed position indicated in Figure 3 with the magnetic attractive forces of the encapsulated magnet 29 reactive to the apertured valve seat 27.

In use, under the magnetic pull from the switching magnet 21, the hinged valve element 28 is pivoted to an open non-occluded position as shown in Figure 4 allowing for free flow of fluid through the valve. In this example, when the switching magnet 21 is brought into close proximity to the valve, with its respective centre line A in or closely aligned to an activation plane defined by the centre line 22A of the valve housing 22 and the perpendicular bisector of the straight portion of the hinge pin 33 at 33A and the closest end of the switching magnet 21 having appropriate magnetic polarity to the outflow side of the magnet 29, indicated by arrows "OF" in Figure 4, then two valve open conditions are possible.

The first valve open position is with the switching magnet 21 on the outflow side of the magnet 29, the magnetic attraction therebetween overcomes that of said valve seat 27.

The second condition for a valve opening is when the switching magnet 21 is on the inlet flow (indicated by arrows "IF") of the magnet 29, the magnetic repulsion exceeds attraction between the magnet 29 and the valve seat 27. Additional force tending to open the valve is provided by magnetic torque induced by the valve element 28 and the misalignment of the switching magnet 21 and the magnet fields of the valve element 28. Thus the switching magnet 21 may be anywhere in the activation plane, but should not approach with its midplane coincident with the midplane of the encapsulated magnet 29 where the effective working distance is least.

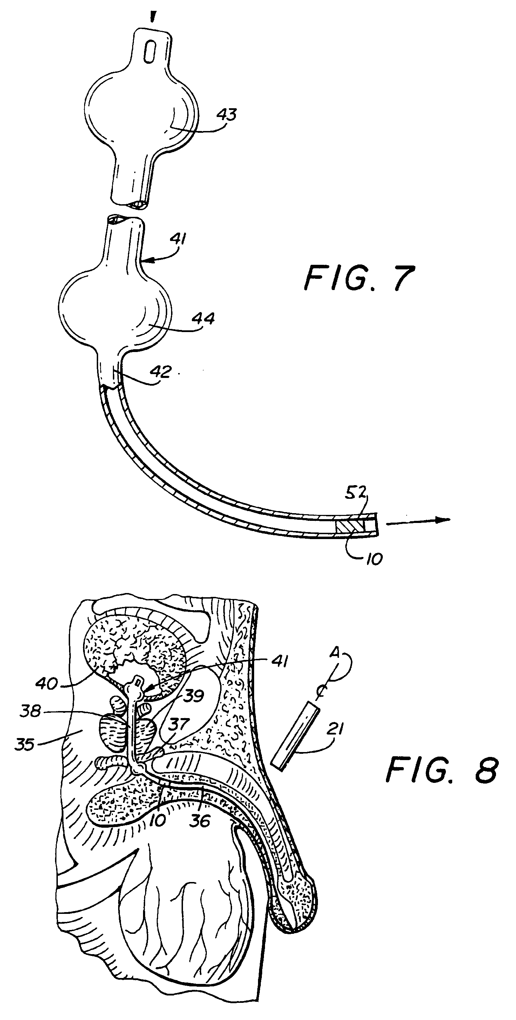

Figure 7 shows an example of a placement vehicle for the valve assembly 10. In this example, chosen for illustration, a modified catheter 41 can be seen having an elongate tubular body member 42 with longitudinally spaced retention bulges 43 and 44 thereon. A portion of the catheter 41 within the prostatic urethra 38 is constructed to resist collapse and be held within by the retaining bulge 43 within the bladder 40 and adjacent the external sphincter 37 as illustrated by the bulge 44 extending as near as possible thereto within the bulbous urethra 36.

The valve 10 is positioned within the catheter 41 adjacent the distal end at 52. The catheter 41 is designed to be easily placed and removed much like known internal catheters.

The valve assembly 10 within the catheter 41 is flow orientated so that the respective valve seats 16 and 27 illustrated are adjacent the upstream flow indicated by the respective inflow arrows IF shown in Figure 4.

In Figure 8, a partial cross-section through a human abdomen 35 illustrates the positioning of the valve assembly 10 within the patient. The abdomen 35 contains the bulbous urethra 36, external sphincter 37, prostatic urethra 38, prostate 39 and bladder 40.

Referring now to Figure 9, another form is illustrated, wherein a cylindrical housing 50 has a valve element 51 within, the valve element 51 being defined by a permanent magnet 46 encapsulated within a coating or plating of biocompatible materials CA adhered to a non-ferromagnetic cylindrical pad 47. The pad 47 is engageable in sealing relation against a valve seat 48 apertured at 49 corresponding to the above described valve seats 16 and 27 shown in respective valve element configurations 18 and 28.

It will be appreciated that the valve assembly 10 can be used in other environments not exclusive to the human body where reliable self-contained remotely actuated valves are required.

## Claims

1. An intraurethral magnetic valve comprising a tubular housing (11, 22, 50) of non-magnetic material, a valve element (18, 28, 51) within said housing and an apertured valve seat (16, 27, 48) sealingly engageable by said valve element, said valve element being normally magnetically attracted to said valve seat thereby occluding and sealing same and there being separate magnetic means (21) for opening said valve by imposing a magnetic field on said valve element to overcome the magnetic attraction to said valve seat thereby moving said valve element away from sealing relation with said valve seat, characterised in that said valve element comprises a permanent magnet (19, 29, 46) and a non-ferromagnetic means (18B, 30, 47) for spacing said permanent magnet with respect to said valve seat, and wherein said moving said valve element away is pivoting said valve element away.

2. An intraurethral magnetic valve according to claim 1 and comprising spaced flanges (13, 26) extending inwardly of said housing and disposed in a spaced relation opposite to said valve seat to retain said valve element within said housing.

3. An intraurethral magnetic valve according to claim 1 or 2, wherein said permanent magnet and said non-ferromagnetic means are cylindrical.

4. An intraurethral magnetic valve according to claim 1, 2 or 3, wherein said non-ferromagnetic means comprises a non-ferromagnetic material of a predetermined thickness secured to said permanent magnet and having a planar surface to provide the fluid seal between said non-ferromagnetic means and said valve seat.

5. An intraurethral magnetic valve according to any one of the preceding claims, wherein said means for opening said magnetic valve comprises a permanent magnet (21) having a longitudinal axis (A) along which it is magnetised.

6. An intraurethral magnetic valve according to any one of the preceding claims, wherein said valve element is hinged within said housing by means of a mounting hinge (32) integral with said valve element and a pivot pin (33) engaging said valve element with said housing.

7. An intraurethral magnetic valve according to any one of claims 1 to 6, wherein said valve element is free to tilt within said housing without fouling sidewalls thereof.

8. An intraurethral magnetic valve according to any one of the preceding claims, wherein said permanent magnet (19, 29, 46) is totally encapsulated.

9. An intraurethral magnetic valve according to claim 8, wherein said encapsulated magnet comprises a biocompatible coating and plating and said means for spacing said permanent magnet (46) with respect to said valve seat (48) comprises a non-ferromagnetic pad (47) secured to said permanent magnet to impart a fluid seal between said magnet and said valve seat.

10. An intraurethral magnetic valve according to any preceding claim and further comprising means for securing said magnetic valve within the urethra of a person, including a contoured placement catheter (41) having elongate tubular body member (42) having portions of longitudinally-spaced retention bulges (43, 44) for positioning and securing said catheter within the person in communication with bladder and urethra.

## Patentansprüche

1. Ein Intraurethralmagnetventil mit einem rohrförmigen Gehäuse (11, 22, 50) aus nichtmagnetischem Material, einem Ventilelement (18, 28, 51) innerhalb des Gehäuses und einem mit einer Öffnung versehenen Ventilsitz (16, 27, 48), der mit dem Ventilelement abdichtend in Anlage bringbar ist, wobei das Ventilelement normalerweise an den Ventilsitz angezogen wird, um hierdurch dieses zu verschließen und abzudichten, wobei eine separate Magnetvorrichtung (21) vorgesehen ist, um das Ventil zu öffnen, in dem ein Magnetfeld auf das Ventilelement aufgebracht wird, um die magnetische Anziehung auf den Ventilsitz zu überwinden, wodurch das Ventilelement aus der abdichtenden Beziehung mit dem Ventilsitz wegbewegt wird, dadurch gekennzeichnet, daß das Ventilelement einen Permanentmagneten (19, 29, 46) und eine nicht-ferromagnetische Vorrichtung (18B, 30, 47) aufweist zum Beabstanden des Permanentmagneten gegenüber dem Ventilsitz, wobei das Wegbewegen des Ventilelementes eine Wegschwenkung des Ventilelementes ist.

2. Ein Intraurethralmagnetventil nach Anspruch 1, mit beabstandeten Flanschen (13, 26), die sich von dem Gehäuse aus nach innen erstrecken und in einer beabstandeten Beziehung gegenüber dem Ventilsitz sind, um das Ventilelement innerhalb des Gehäuses zu halten.

3. Ein Intraurethralmagnetventil nach Anspruch 1 oder 2, wobei der Permanentmagnet und die nicht-ferromagnetische Vorrichtung zylindrisch sind.

4. Ein Intraurethralmagnetventil nach Anspruch 1, 2 oder 3, wobei die nicht-ferromagnetische Vorrichtung ein nicht-ferromagnetisches Material bestimmter Dicke aufweist, welches an dem Permanentmagneten festgelegt ist und eine ebene Oberfläche hat, um die Fluidabdichtung zwischen der nicht-ferromagnetischen Vorrichtung und dem Ventilsitz bereitzustellen.

5. Ein Intraurethralmagnetventil nach einem der vorhergehenden Ansprüche, wobei die Vorrichtung zum Öffnen des Magnetventils einen Permanentmagneten (21) aufweist mit einer Längsachse (A), entlang der dieser magnetisiert ist.

6. Ein Intraurethralmagnetventil nach einem der vorhergehenden Ansprüche, wobei das Ventilelement innerhalb des Gehäuses mittels eines Befestigungslagers (32) angelenkt ist, welches einstückig mit dem Ventilelement ist, sowie durch einen Schwenkzapfen (33), der das Ventilelement mit dem Gehäuse in Eingriff hält.

7. Ein Intraurethralmagnetventil nach einem der Ansprüche 1 bis 6, wobei das Ventilelement innerhalb des Gehäuses frei verkippen kann, ohne die Seitenwände hiervon zu berühren.

8. Ein Intraurethralmagnetventil nach einem der vorhergehenden Ansprüche, wobei der Permanentmagnet (19, 29, 46) vollständig gekapselt ist.

9. Ein Intraurethralmagnetventil nach Anspruch 8, wobei der gekapselte Magnet einen biokompatiblen Überzug und eine ebensolche Beschichtung aufweist, und wobei die Vorrichtung zum Beabstanden des Permanentmagneten (46) von dem Ventilsitz (48) ein nicht-ferromagnetisches Kissen (47) aufweist, welches an dem Permanentmagneten festgelegt ist, um zwischen dem Magneten und dem Ventilsitz eine Fluidabdichtung zu erzeugen.

10. Ein Intraurethralmagnetventil nach einem der vorhergehenden Ansprüche, weiterhin mit einer Vorrichtung zum Festlegen des Magnetventiles innerhalb der Harnröhre einer Person, mit einem konturierten Katheter (41) mit einem langgestreckten rohrförmigen Körper (42), der Abschnitte mit in Längsrichtung beabstandeten Halteausbauchungen (43, 44) hat, um den Katheter innerhalb der Person in Verbindung mit Harnblase und Harnröhre zu positionieren und festzulegen.

## Revendications

1. Soupape magnétique intra-urétrale comprenant un logement tubulaire (11, 22, 50) en matériau non magnétique, un élément de soupape (18, 28, 51) situé à l'intérieur dudit logement et un siège de soupape à ouverture (16, 27, 48) pouvant être engagé de façon hermétique avec ledit élément de soupape, ledit élément de soupape étant attiré magnétiquement perpendiculairement audit siège de soupape de façon à obturer et à fermer hermétiquement celui-ci et placés là, des moyens magnétiques séparés (21) pour ouvrir ladite soupape en imposant un champ magnétique sur ledit élément de soupape en vue de surmonter l'attraction magnétique exercée sur ledit siège de soupape de façon à déplacer ledit élément de soupape hors de l'étanchéité avec ledit siège de soupape, caractérisée en ce que ledit élément de soupape comprend un aimant permanent (19, 29, 46) et des moyens non ferromagnétiques (18B, 30, 47) pour séparer ledit aimant permanent dudit siège de soupape, et dans laquelle ledit élément de soupape écarté pivote à distance dudit élément de soupape.

2. Soupape magnétique intra-urétrale selon la revendication 1 et comprenant des rebords espacés (13, 26) s'étendant vers l'intérieur dudit logement et disposés de façon distante à l'opposé dudit siège de soupape afin de retenir ledit élément de soupape à l'intérieur dudit logement.

3. Soupape magnétique intra-urétrale selon la revendication 1 ou 2, dans lequel ledit aimant permanent et lesdits moyens ferromagnétiques sont cylindriques.

4. Soupape magnétique intra-urétrale selon la revendication 1, 2 ou 3, dans laquelle lesdits moyens non ferromagnétiques comprennent un matériau non ferromagnétique d'une épaisseur prédéterminée fixé audit aimant permanent et présentant une surface plane pour fournir l'étanchéité au fluide entre lesdits moyens non ferromagnétiques et ledit siège de soupape.

5. Soupape magnétique intra-urétrale selon l'une quelconque des revendications précédentes, dans laquelle lesdits moyens pour ouvrir ladite soupape magnétique comprennent un aimant permanent (21) comportant un axe longitudinal (A) suivant lequel il est aimanté sur sa longueur.

6. Soupape magnétique intra-urétrale selon l'une quelconque des revendications précédentes, dans laquelle ledit élément de soupape est articulé à l'intérieur dudit logement au moyen d'une articulation de montage (32) intégrée audit élément de soupape et une broche de pivotement (33) engageant ledit élément de soupape avec ledit logement.

7. Soupape magnétique intra-urétrale selon l'une quelconque des revendications 1 à 6, dans laquelle ledit élément de soupape est libre de basculer à l'intérieur dudit logement sans heurter ses parois latérales.

8. Soupape magnétique intra-urétrale selon l'une quelconque des revendications précédentes, dans laquelle ledit aimant permanent (19, 29, 46) est complètement encapsulé.

9. Soupape magnétique intra-urétrale selon la revendication 8, dans laquelle ledit aimant encapsulé comprend un revêtement et un placage biocompatibles et lesdits moyens servant à éloigner ledit aimant permanent (46) dudit siège de soupape (48) comprennent un patin non ferromagnétique (47) fixé audit aimant permanent pour obtenir une étanchéité au fluide entre ledit aimant et ledit siège de soupape.

10. Soupape magnétique intra-urétrale selon l'une quelconque des revendications précédentes et comprenant, de plus, des moyens pour fixer ladite soupape magnétique à l'intérieur de l'urètre d'une personne, comprenant un cathéter de placement profilé (41) présentant un corps tubulaire de forme allongée (42) comportant des parties renflées de rétention espacées longitudinalement (43, 44) pour positionner et fixer ledit cathéter à l'intérieur de la personne en communication avec la vessie et l'urètre.
